# EUROPEAN PATENT APPLICATION

(11) **EP 4 275 790 A1**
(43) Date of publication of application: **15.11.2023**
(21) Application number: 22172234.1
(22) Date of filing: 09.05.2022
(51) Int. Cl.: B01J 29/78, B01J 29/70, B01J 21/18, B01J 21/06, B01J 23/34, B01J 35/00, B01J 37/04, A61L 9/20, F24F 8/167, B01D 53/86

(54) **CATALYTIC COMPOSITION AND CATALYTIC DEVICE**

(71) Applicant: Calistair SAS, 77000 Vaux-le-Pénil (FR)
(72) Inventor: Taranto, Jérôme, 77000 Vaux-le-Pénil (FR); Carvalho, Alexandre, 77000 Vaux-le-Pénil (FR); Golamaully, Azdeen, 77000 Vaux-le-Pénil (FR); Hachet, Alain, 77000 Vaux-le-Pénil (FR)
(74) Representative: Kröncke, Rolf

(57) **Abstract**

The present invention relates in a first aspect to a catalytic composition comprising a catalytic mixture in a powdered state of a first catalyst having photo catalytic activity, a second catalyst being a low temperature catalyst and an adsorbent, whereby the catalytic composition comprises carbon nanotubes in an amount of at most 5 weight-% based on the amount of the catalytic mixture. Further, the present invention relates to a method for producing said catalytic composition according to the present invention. In addition, a catalytic device comprising the catalytic composition according to the present invention, optionally coated on a carrier is provided as well as a gas depolluting apparatus containing said catalytic composition according to the present invention or the catalytic device accordingly. Finally, the use of the catalytic composition or the catalytic device as well as the gas depollution apparatus is disclosed, in particular, for depollution of gases of volatile chemical contaminants as well as treatment of gas containing biological contaminants.

## Description

The present invention relates in a first aspect to a catalytic composition comprising a catalytic mixture in a powdered state of a first catalyst having photocatalytic activity, a second catalyst being a low temperature catalyst and an adsorbent, whereby the catalytic composition comprises carbon nanotubes in an amount of at most 5 weight-% based on the amount of the catalytic mixture. Further, the present invention relates to a method for producing said catalytic composition according to the present invention. In addition, a catalytic device comprising the catalytic composition according to the present invention, optionally coated on a carrier is provided as well as a gas depolluting apparatus containing said catalytic composition according to the present invention or the catalytic device accordingly. Finally, the use of the catalytic composition or the catalytic device as well as the gas depollution apparatus is disclosed, in particular, for depollution of gases of volatile chemical contaminants as well as treatment of gas containing biological contaminants.

### Prior Art

The depollution of gases is widely used and needed. Contaminants within the gases, especially within air, can be of various types. They can be divided into particles, gaseous or volatile chemical contaminants as well as biological contaminants. The biological contaminants contain all types of microorganisms, such as bacteria, fungi including their spores or mites but also viruses, phages and the like.

As said, gaseous or volatile substances contain a variety of different types of compounds, including the Volatile Organic Compounds (VOC) as well as Very Volatile Organic Compounds (WOC). Typically, these types of volatile compounds are termed also Total Volatile Organic Compounds (TVOC) accordingly. Of note, inorganic compounds like carbon oxides as well as other inorganic carbonates are not included into this definition, but organic compounds only.

Various possibilities to treat and depollute gas, especially air, is known in the art. Starting with the use of mechanical filters, such as HEPA filters or the like, particulate contaminants can be filtered off, these particulate contaminants include particulate chemical but also and importantly biological contaminants. However, these filtering units only filter off the particulate components or contaminants without destroying the same. In addition, these filters need to be cleaned and replaced after a certain period of time. Moreover, depending on the pore size of the filters but also other parameters like air velocity, electrostatic forces and Brownian motion. These filters are less efficient towards particles having a size below 1 µm, like for adsorbing viruses. Moreover, since the biological contaminants are not inactivated or destroyed, harmful substances may be released into the gas flow leaving the filter system. For example, toxic substances can be endotoxins formed after lysis of some gramnegative bacteria cell walls or VOC from microorganism decomposition on filters. Vulnerable microorganisms such as *Staphylococcus epidermidis, E. coli* or *Brevundimonas diminuta* can survive for two to six days on such a filter. Even more robust biological contaminants such as *Bacillus atrophaeus, MS-2 Coliphage,* and *Aspergillus brasilensis* survive more than six days. It is described in the art that e. g. *bacillus atrophaeus* can remain viable for more than 210 days. Accordingly, the mechanical separation of contaminants is not sufficient. Moreover, the contaminants, in particular, biological contaminants can lead to a saturation of the filter leading to an increased flow resistance. This is particularly not desirable if the filter system is present in respiratory or breathing masks as well as gas masks. In particular, saturation of the filter by biological contaminants which are filtered and afterwards additionally colonizing the filter surface, can lead to a general decrease of the performance of a gas depolluting apparatus. Inter alia, problems occurring are odours, a decrease of the air flow and an increase of the energy consumption and the formation of biofilms of internal surfaces of the filter and the gas depolluting apparatus in general. Nevertheless, such filters can be useful to eliminate the contained contaminants.

In addition, a further method to remove chemical pollutants from gases is the adsorption, in particular, for indoor air treatment. Typical adsorbents used are activated carbon and zeolites to adsorb and trap the molecules accordingly.

Other solutions for air depollution and/or purification include the use of plasma, ionization, ozonization or catalysis.

Catalysts are used in many industrial fields. A catalyst is a substance which is able to catalyse reactions, namely, to increase the rate of a chemical reaction without being consumed in the reaction. One specific field is the use of catalysts for depolluting gases, such as air, especially ambient air, or exhaust process gases. Various forms of applications of these catalysts are known, including air conditioning systems as well as air purifying systems. Catalysts may include catalysts having a photocatalytic activity, also referred to as photocatalysts, usually being semiconductors. Typically, the photocatalyst is UV activatable. Namely, when UV activatable photocatalysts are irradiated with UV radiation, it is then activated and therefore will catalyse reactions. In this connection, UV radiation refers to electromagnetic radiation with wavelengths of about 100 nm to about 400 nm.

Reactive radical species generated on the surface of the catalyst react with the contaminants in the passing gas flow, destroying e. g. lipid layer of the microorganisms then their nucleic acids (DNA or RNA). Moreover, a catalytic system may comprise different types of catalysts being combined at different stages in the flow direction of the gas to be depolluted. Also admixes of catalytic compositions, particularly useful for gas depollution, are known.

For example, WO 2021/130388 A1 identifies a method for manufacturing a photocatalytic device, photocatalytic composition and gas depolluting apparatus containing said catalytic composition and catalytic devices accordingly. Therein, a catalytic composition comprising a photocatalyst, a second catalyst being a low temperature catalyst and an adsorbent are described. Namely, the catalytic mixture described therein is coated on carriers whereby preferably said catalytic mixture is composed of TiO₂, MnO and zeolite.

Also JPH11-137656 discloses a catalytic composition, consisting of manganese oxide, titanium dioxide and an adsorbent, whereby these are bound to a solid support via a binder. However, the binder binder partly covers the catalysts and therefore only a reduced amount can be activated by UV radiation and, in addition, the catalytic activity is decreased in view of the binder. In contrast, the catalytic composition disclosed in WO 2021/130388 A1 is coated on a solid grid-like carrier by applying a suspension liquid of the admixture and coating the same on said carrier repeatedly.

Although the catalytic system and devices described therein are useful for gas depollution, it is desired to improve these systems further.

Accordingly, the aim of the present invention is to provide new catalytic compositions, particularly useful for gas depollution as well as methods for producing the same, and catalytic devices and gas depolluting apparatus accordingly.

Carbon nanotubes (CNTs) have been applied in a diverse range of industrial and research processes, such as energy storage, hydrogen production, gas separation and absorption, photocatalytic and catalytic processes. The CNTs can be considered as a cylinder created by curling a graphene sheet having a rectangular hexagon structure. Based on the number of graphene surface layers, they are categorized into single-walled carbon nanotubes (SWCNTs), having one graphene sheet and multi-walled carbon nanotubes (MWCNTs), displaying several graphene sheets.

It is described in the field of photocatalysis, that the unique electronic properties of the CNTs lead to the improvement of the efficiency of commercial photocatalysts. Various publications report on the use of CNTs as a catalyst also in the field of gas treatment. For example, CN 109908883 discloses a method to prepare a nanocomposite based on nanocarbon and metal oxide for photoelectronic catalytic processes with microporous carbon material including CNTs and graphene as a matrix. An organic slurry was mixed with metal oxide particles to obtain a nanometal oxide film. Then the matrix was coated with the nanometal oxide film and treated further to remove the solvent and attaching the metal oxide particles to the CNT graphene structure. It is discussed that the material can improve organic gas removal efficiency by photocatalysis. Typically, the CNTs are combined with other catalytic materials by sol-gel methods known to the skilled person.

### Brief description of the present invention

In a first aspect, the present invention relates to a catalytic composition comprising:
a) A catalytic mixture in a powdered state of i) a first catalyst having photocatalytic activity, ii) a second catalyst being a low-temperature catalyst, and iii) an adsorbent, and
b) Carbon nanotubes (CNT) in an amount of at most 5 weight-% based on the amount of the catalytic mixture of a).

In particular, the catalytic composition is a composition wherein the catalytic mixture is in a powdered state comprising in weight-% in regard to its total mass
between 27 % and 30 % of photoactivated TiO₂,
between 11 % and 17 % of MnO,
between 55 % and 59 % of zeolite, in particular, hydrophilic zeolite of type A, and
between 0.1 % to 2 % based on the total amount of the catalytic mixture of carbon nanotubes.

Moreover, a method for producing a catalytic composition is provided. In a further aspect, the present invention relates to a catalytic composition obtainable by the method according to the present invention as well as a catalytic device comprising the catalytic composition according to the present invention, optionally coated on a carrier.

Further, a gas depolluting apparatus comprising the catalytic device according to the present invention or a catalytic composition according to the present invention is provided, wherein the catalytic device or the catalytic composition is at least partly provided with a designated flow path of the gas to be depolluted.

Finally, the present invention relates to the use of a catalytic composition according to the present invention or obtainable by the method according to the present invention or to the use of the gas depolluting device according to the present invention or the gas depolluting apparatus according to the present invention in a method for depollution of gaseous or volatile chemical contaminants and/or the treatment of gas containing biological contaminants.

### Brief description of the drawings

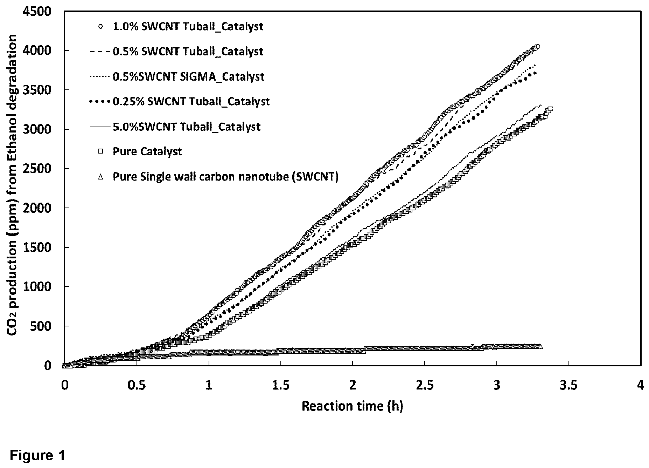

- Figure 1:: CO₂ production from the catalytic degradation of ethanol as a function of reaction time.

### Detailed description of the present invention

The present inventors recognized that a catalytic composition containing the catalytic mixture described in WO 2021/191307 in combination with small amounts of carbon nanotubes (CNTs), in particular, CNTs in an amount of at most 5 weight-% based on the amount of the catalytic mixture, allows improved photocatalytic degradation of chemical contaminants based on the model contaminant ethanol. Moreover, the catalytic composition is suitable for depollution of biological contaminants as well. In particular, it has been recognized that degradation process can be performed faster when admixing in low amounts CNTs to the catalytic mixture.

Beside the described combination of high depollution efficiency and at the same time on microbial growth with the catalytic mixture in the first catalyst having photocatalytic activity, the second catalyst being a low temperature catalyst and the adsorbent, the low amounts of CNTs increases the degradation time of the contaminants accordingly.

In an embodiment, the first catalyst and the second catalyst are different molecules. That is, the first catalyst and the second catalyst are different in their chemical composition. Namely, the first catalyst and the second catalyst cannot be identical compounds having different properties, including different particle sizes, crystal structures, shapes or the like. For example, the first catalyst and the second catalyst cannot be TiO₂ in various particulate forms, structures or anything like this, but are chemically different. In other words, a composition comprising one compound in different shapes, crystal structures or the like, cannot be considered as comprising two different catalysts as claimed herein.

A catalyst having photocatalytic activity is a substance that is factually capable of being photoactivated by irradiation, for examples, as specified below. A substance having photocatalytic properties which is not capable of being photoactivated for example due to being embedded in a matrix or coated, thus, being not available for photoactivation, is not considered a catalyst having photocatalytic activity. A typical example of a suitable catalyst is titanium dioxide. Titanium dioxide is known for various application, as a scattering agent in the cosmetic industry. Typically, this titanium dioxide used in the cosmetic industry is treated to suppress the photocatalytic activity, e. g. by coating. In most applications, the photocatalytic activity of these particles, TiO₂ particles, would lead to the regeneration of ROS (Reactive Oxygen Species), which is undesired and harmful in cosmetics. Consequently, coated titanium dioxide particles do not catalysts having photocatalytic activity since they cannot be factually photoactivated. This also accounts for such articles embedded in matrixes which does not allow radiation to reach the particles and, thus, to photoactivate these particles. The catalytic mixture is present in a powdered state. The same is true for the CNTs mixed thereto.

In an embodiment, the CRT are selected from multi-walled carbon nanotubes (MWCNTs) or single-walled carbon nanotubes (SWCNTs).

The skilled person is well aware of suitable types of carbon nanotubes for use in the catalytic composition. The first catalyst, the second catalyst and the adsorbent are provided as distinct powders and mixed, e. g. by mingling, to obtained a catalytic mixture. This catalytic mixture is then mixed further with the carbon nanotubes to obtain the catalytic composition according to the present invention.

Only after mixing the catalytic mixture with the carbon nanotubes, further suspension in a suspension liquid may be conducted for later coating of a carrier.

In the catalytic composition, the first catalyst, for example can contain tungsten oxide and/or zinc oxide. In a preferred embodiment, the first catalyst is titanium dioxide TiO₂. Of course, not only one type of first catalyst can be used, but it is also possible to use more than one catalyst having photocatalytic activity. The titanium dioxide is well known for its photocatalytic properties. It is a single conductor and can be activated by irradiation with UV radiation of a wavelength from 100 nm to 400 nm. Preferably, the UV radiation used for activating the first catalyst, especially titanium dioxide, has wavelengths of 365 nm or less. In an aspect, it is preferred that the UV radiation is in the UV-C range, between 100 and 280 nm, like 254 nm. When using UV radiation in UV-C range, the direct disinfectant properties of the UV-C radiation can be used advantageously. That is, the UV-C radiation can act directly biocidal due to its high energy and indirect biocidal on known biological contaminants by activating the first catalyst. In another aspect, the UV radiation is an UV-A radiation. This is particularly true for chemical contaminants, like VOC contaminants. The titanium dioxide can be already photoactivated. Titanium dioxide can be applied cost efficiently and has the ability to at least partly regenerate itself. Further, it is highly active against different contaminants, including chemical and biological contaminants. For example, the titanium dioxide may be used in its crystalline form of anatase as well as admixtures with rutile. For example, the first catalyst being a titanium dioxide may be in form of a mixture of anatase and rutile with an anatase/rutile ration of 60/40 or 90/10. For example, the range is at least 60/40, preferably, at least 70/30, especially at least 80/20. The ratio may not exceed 90/10, preferably not exceeding 95/5 and especially not exceeding 90/10. For example, the ratio is in between 70/30 and 83/17, especially 80/20.

In another embodiment, the catalyst may be a 100/0 (anatase/rutile ration) catalyst as a photocatalyst according to the present invention.

The first catalyst may be doped with further ions, like silver ions or platinum ions. The presence of the doping ions increases the number of possible oxidation reduction reactions. The titanium dioxide preferably has an elementary particle size of 10 to 50 nm, more preferably of 10 to 35 nm, especially around 25 nm. Since the elementary particles tend to aggregate, the particle size of these aggregates is preferably in the range of in between 200 and 600 nm, more preferably between 300 and 500 nm, especially around 420 nm.

The second catalyst is a low temperature catalyst. Low temperature catalysts activated by calorific energy. In particular, a low temperature catalyst is a catalyst being defined as manganese oxide (MnO) activated by a relative low temperature (between 20 and 80°C).

The term low temperature catalyst is hereby used to differentiate the type of catalyst from thermal catalyst, which activates at relative high temperatures of usually 500 °C to 1200 °C. The low temperature catalyst according to the present invention is not only catalytically active at a relatively low temperature, but is activated by light and relatively low temperature. For clarification, a photocatalyst which is capable of being photoactivated at ambient temperature is no low temperature catalyst, because it is not activated by the calorific energy of the ambient temperature, but by the radiation. A low temperature catalyst preferably is a catalyst already being catalytically activated and, thus, active at temperatures lower than 100 °C, more preferably at temperatures lower than 50 °C, most preferably already at room temperature of 20 °C. Of course, at high temperatures the catalyst may be active as well. Low temperature catalysts are for example metal oxides, like nickel oxide or cerium oxide. In a preferred embodiment of the invention, the second catalyst is manganese oxide, being an efficient low temperature catalyst. In an embodiment, the manganese monoxide is not or substantially not contaminated with manganese dioxide, e. g. the amount of manganese dioxide is lower than 5 %, preferably lower than 1 %, most preferably lower than 0.1 % of the total mass of manganese monoxide used as second catalyst. In an embodiment, no manganese dioxide is present. The manganese monoxide, e. g. in crystalline form, allows the generation of highly reactive radical species when in contact with oxygen, e. g. present in air. Preferably, the temperature is higher than 35 °C, like in between 35 °C and 55 °C, more preferably between 45 °C and 50 °C.

In an embodiment, the relative humidity of the gas to be depolluted, like air, is between 30 % and 80 %, preferably 50 %. This allows very efficient generation of radical species.

In case the second catalyst is manganese monoxide, its average particle size is preferably between 50 nm and 170 nm, like between 95 nm and 135 nm, e. g. around 110 nm.

The adsorbent is preferably a compound having a large specific surface area, preferably of at least 300 m²/g, more preferably of at least 500 m²/g, most preferably of at least 1,000 m²/g, in particular, more than 2,000 m²/g. The adsorbent can for example be activated carbon.

In a preferred embodiment, the adsorbent is a zeolite. Zeolite are microporous aluminium silicate minerals which can be naturally occurring or being prepared artificially. Preferably a hydrophilic zeolite is used, preferred embodiments include a zeolite of type A or type ZSM-5. The zeolite is preferably a synthetic zeolite, especially a synthetic zeolite of type A or ZSM-5.

As said, the catalytic composition according to the present invention comprises in an embodiment a catalytic mixture in a powdered form comprising in weight-% with regard to the total mass of the catalytic mixture:
between 20 % to 65 % of the first catalyst having photocatalytic activity,
between 5 % to 55 % of the second catalyst having low-temperature activity, being different to the first catalyst, and between 30 % to 70 % of an adsorbent. The ratio between the zeolite of type A and titanium dioxide typically ranges between 3:1 and 1:1, especially as around 2:1. The ratio of the zeolite of type A and manganese monoxide preferably ranges around 5:1 and 3:1, especially is around 4:1. The ratio of titanium dioxide and manganese monoxide preferably ranges between 3:1 and 1:1, especially is around 2:1. The different types of ratios are also true for other catalysts and adsorbents used according to the present invention. Preferably, the zeolite, especially the zeolite of type A, comprises a sodalite crystal structure. The amount of the first catalyst preferably is at least 20 % and does preferably not exceed 65 %, like does not exceed 40 %, in particular, does not exceed 30 % of the total mass of the catalytic mixture composed of the first catalyst, the second catalyst and the adsorbent. The amount of the second catalyst is at least 5 %, more preferably at least 10 %, like at least 15 % and does preferably not exceed 55 %, like does not exceed 30 %, in particular, does not exceed 20 % of the total mass of the first catalyst, the second catalyst and the adsorbent.

Further, the adsorbent is preferably in a range of 30 % to 70 %, like at least 40 %, in particular, at least 50 %. Further, the adsorbent may not exceed 70 weight-%, like 60 weight-%.

Moreover, the carbon nanotubes are typically present in at most 5 weight-% based on the total amount of the catalytic mixture. In an embodiment, the carbon nanotubes are present in an amount of at most 4 weight-%, like at most 3 weight-%. In an embodiment, at least 0.05 weight-%, like 0.1 weight-% of the carbon nanotubes is present in the catalytic composition accordingly.

In an embodiment, the powdered catalytic mixture present in the catalytic composition comprises in weight-% in regard to its total mass
between 27 % and 30 % of photoactivated TiO₂,
between 11 % and 17 % of MnO,
between 55 % and 59 % of zeolite, in particular, hydrophilic zeolite of type A, and
15 between 0.1 % to 2 % based on the total amount of the catalytic mixture of carbon nanotubes.

In a further aspect, a method for reducing the catalytic composition containing a first catalyst having photocatalytic activity, a second catalyst different to the first catalyst being a low-temperature catalyst, an adsorbent present in form of a catalytic mixture, and carbon nanotubes comprising the steps of
a) Preparing a catalytic mixture composed of the first catalyst, the second catalyst and the adsorbent, whereby each of the components are provided in a powdered form, including mingling the same to obtain particles of the catalytic mixtures;
b) Admixing the particles of the catalytic mixture obtained in step a) with carbon nanotubes to obtain the catalytic composition
is provided.

According to the method for producing the catalytic composition according to the present invention, firstly the catalytic mixture is prepared by mixing the powdered components of the catalytic mixture, namely, the first catalyst, the second catalyst and the adsorbent.

After obtaining the mixture of the catalytic mixture according to the present invention, the carbon nanotubes are added to said mixture in a powdered form.

That is, in an embodiment, a solvent free mixture is applied to obtain the mixture of the catalytic composition admixing the catalytic mixture with the carbon nanotubes accordingly.

Namely, by simple mechanical mixing the carbon nanotubes are admixed with the catalytic mixture to obtain the catalytic composition according to the present invention. That is, in an embodiment, the mixing of the CNTs with the particles of the catalytic mixture is by mingling the components in powdered, dry form. Since the mixture of the catalytic mixture composed of the first catalyst, the second catalyst and the adsorbent are present in large aggregates, the powdered small CNTs adhere to these larger aggregates accordingly.

In another embodiment, the process for obtaining the catalytic composition according to the present invention includes a wet mixing composed of a two-step mixing method. Namely, this method comprises the first step of mechanically mixing the catalytic mixture with the carbon nanotubes to obtain a powdered, catalytic composition according to the present invention. Subsequently, solvent is added under agitation, optionally with heat transfer, allowing dispersion of the composite or catalytic composition comprising the catalytic mixture and the carbon nanotubes. The composite is dispersed further under e. g. ultrasonic agitation at elevated temperature. After dispersion, the solvent is evaporated eventually obtaining the catalytic composition powder accordingly.

Each of the components of the catalytic mixture are prepared by known methods, e. g. as described in WO 2021/130388. The disclosure of WO 2021/130388 is enclosed herewith by reference fully.

The catalytic composition according to the present invention is coated to a carrier to obtain a catalytic composition by known means. For example, suitable coating procedures are described in WO 2021/130388.

In particular, in view of the CNT it is preferred to use suitable solvents or mixture of solvents to disperse the catalyst containing the CNT. Suitable catalysts coatings include ethanol, methanol, isopropanol, propanol, acetone or DMF.

Suitable carriers for the catalytic device having the catalytic composition according to the present invention coated thereon include known carrier materials for catalysis, e. g. when present in gas depolluting apparatus, the carrier is a solid grid-like carrier having a plurality of through holes configured to allow a gas to flow through the carrier. The carrier can for example be formed from a metal, such as aluminium or steel, a plastic material or a composite material. It can for example be formed as a grid, a plate or an expanded metal. In an embodiment of the present invention, the carrier is shaped to a corrugated and/or folded sheet forming a plurality of through holes. In an embodiment, the carrier is formed in a honeycomb manner. Advantages of a honeycomb layout compared to a plane material are a larger surface to be coated and a lower pressure draw. Also the irradiance within the cells is better.

The carrier then often simply referred to as a honeycomb or honeycomb panel or honeycomb carrier. Preferably, the through holes have a hexagonal cross-section. The coating of the catalytic composition according to the present invention has preferably a total thickness of at least 50 µm, more preferably at least 75 µm, especially 100 µm. The coating of such thickness especially provides a satisfactory amount of catalysts for gas depollution. Preferably, the thickness does not exceed 500 µm, preferably does not exceed 350 µm, and especially does not exceed 250 µm. The preferred range lies between 100 µm and 250 µm. It is desired that the coating resists an air pressure of up to 4 bar. The slurry of the catalytic composition according to the present invention can be applied to the carrier by various methods including dipping. In an embodiment, the slurry is applied by a spray coating, e. g. by a spray gun. Typically, the slurry applied to the carrier is binder free.

The slurry is typically an aqueous suspension. An aqueous solution or *an* organic solvent can be applied to disperse the catalyst. In addition, production containing CNT can be find in aqueous solution with surfactants Since there are different types of surfactants, it will be necessary to evaluate the effect of the surfactant on the reaction. In another aspect, the present invention provides a gas depolluting apparatus, comprising a catalytic device according to the present invention and/or a catalytic composition according to the present invention, whereby the catalytic device or the catalytic composition is at least partly provided within a designated flow path of the gas to be depolluted. The gas depollution apparatus comprises a flow path which is for example determined by a pipe or tube or the housing of the gas depolluting apparatus, especially an inner wall of the housing. The gas to be depolluted gets inside the gas depolluting apparatus through an entry opening and flows through the apparatus along the flow path and exits the apparatus through an exit opening. For example, an entry filter may be present at the entry opening or nearby before the catalytic device and/or the catalytic composition in the direction of flow. That is, in an embodiment, the gas depolluting apparatus comprises a particulate filter as entry filter accordingly. The skilled person is well aware of suitable designs for gas depolluting apparatus using a catalytic composition or a catalytic device according to the present invention for depolluting the gas flow from gases or liquid chemical contaminants and biological contaminants, in particular, breaking these contaminants but also destroying microorganisms or viruses. The gas depolluting apparatus preferably contains more than one treatment stages. Further reference is made to the gas depolluting apparatus described in WO 2021/130388 which is incorporated by reference fully. The gas depolluting apparatus comprises at least one or more radiation sources to activate the photocatalysts of the catalytic device and/or the catalytic composition.

The gas depolluting apparatus can be used for various applications. It can for example be used to depollute air in hospitals, preferably, in operation theatres or quarantine stations. Furthermore, for depollution of gaseous or volatile chemical contaminants or biological contaminants, for example, also in the field of CBRN defence, the catalytic composition and the catalytic device according to the present application can be useful. As described in the examples, the catalytic composition allows fast and efficient decomposition and degradation of contaminants.

The invention will be described further by reference to the examples without limiting the same thereto.

### Examples

### Materials and methods

### Preparation of composites

The single wall carbon nanotubes (SWCNT)- Catalyst composite was prepared by physical mixture and wet mixture (data not shown). The catalytic mixture consist of: photocatalytic TiO₂, low temperature catalytic MnO and zeolite sorbent. In the physical mixture method, the nano-sized SWCNT particles was attached to the relatively large sized particles of the catalyst. It is submitted that since the size of the guest particles is small, van der Waals interactions can be strong enough to hold them to the catalyst host particles.

The wet mixture is a two-step mixing method with the first one being the physical mixture of the SWCNT into catalyst powder to subsequent solvent addition under agitation and with heat transfer. In this case, the ethanol aqueous solution 80%(v/v) was added to disperse the composite under ultrasonic agitation at 80° C. The evaporation was performed under agitation to obtain the final SWCNT-Catalyst powder.

The two procedures were applied to elucidate the effect of synthesis conditions on the photocatalytic performance. The catalyst was provided by the inventors from CALISTAIR SAS company. OCSial and Sigma-Aldrich (Brand 2) were the sources of the SWCNT material.

The following terminology is employed to identify the composites prepared:
x SWCNT Brand_Catalyst
where x is the percentage of SWCNT in weight.

### Catalytic performance

The catalytic degradation of ethanol was performed in a top-irradiation Pyrex reactor (750 cm3 volume and 6.0 cm diameter) under UV light (λ = 375 nm, three lamps of 9.0 W) at ambient temperature (20 - 22°C). In each test, 40 mg of the sample supported on a glassfiber inert material was loaded in the reactor followed by aeration with high-purity air for 30 min to remove CO₂. A pump with heating system injected 4.4 µL of ethanol diluted in dried air into the Pyrex reactor. The kinetics of the CO₂ formation trough ethanol conversion was analyzed by CO₂ analyzer Quantex Model 906 instrument.

The catalytic degradation of ethanol was performed as model reaction to determine the efficiency of the samples (composites and bare materials). Figure 1 shows the ethanol degradation of different samples. The control test (ethanol injection and under irradiation) demonstrated the non-CO₂ production from the experimental system.

The 0.25% SWCNT Tuball_Catalyst enhanced the kinetics of ethanol degradation (45% in 1 h reaction) when compared with pure catalyst. Interesting, the SWCNT concentration from 0.25wt.% to 0.5wt.% and 1.0 wt.% SWCNT resulted in an even faster photocatalytic ethanol degradation in comparison with bare LTP. The 0.5 wt.% SWCNT Tuball_Catalyst and 1.0 wt.% SWCNT Tuball Catalyst composites exhibited higher carbon dioxide production (80% in 1 h). Likewise, the application of a SWCNT from Sigma at 0.5% resulted in a higher kinetics than the pure catalyst material.

A higher amount of SWCNT mechanically mixed to the catalyst (5.0 w.t%) had no significant impact on the efficiency. Such result indicates the existence of an upper limit of SWCNT addition in the catalyst material applied in the present work.

The bare SWCNT was not capable to perform the reaction. It is disclosed that - catalyst material is required to perform the ethanol degradation. Hence, the mixing of SWCNT with catalyst valorizes the carbon-based material.

To conclude, the patent discloses the preparation and the performance of CNT, here, a single wall carbon nanotube (SWCNT)-Catalyst composite. An environmentally friendly and economical feasible method was applied to obtain the SWCNT-Catalyst composite. The mechanical method was able to modify the solid phase reactivity of the Catalyst thus creating a new composite material. Higher ethanol degradation efficiency over the SWCNT-Catalyst composite in comparison with bare Catalyst material is demonstrated.

Two procedures (physical mixture and wet mixture) were applied to obtain SWCNT-Catalyst composites. In particular, the physical mixture was able to form SWCNT-Catalyst composites displaying higher kinetics of ethanol degradation when compared to bare catalyst material. The method does not require a drying step which results in major energy savings. This is an important requirement in terms of SWCNT application in air-treatment processes since the material itself displays high market value. Therefore, small SWCNT (0.25 wt.% to 1.0 wt.%) addition is required to preserve the economically feasibility of the photocatalytic process. Moreover, the method of preparation applied is environmentally benign since do not produce organic or aqueous waste.

## Claims

1. Catalytic composition comprising:
a) A catalytic mixture in a powdered state of i) a first catalyst having photocatalytic activity, ii) a second catalyst being a low-temperature catalyst, and iii) an adsorbent, and
b) Carbon nanotubes (CNT) in an amount of at most 5 weight-% based on the amount of the catalytic mixture of a).

2. Catalytic composition according to claim 1, wherein the carbon nanotubes are selected from multi walled carbon nanotubes (MWCNT) or single wall carbon nanotubes (SWCNT).

3. The catalytic composition according to claim 1 or 2, wherein the first catalyst is titanium dioxide TiO₂.

4. The catalytic composition according to any one of the preceding claims, wherein the adsorbent is a zeolite, in particular, a hydrophilic zeolite of type A.

5. The catalytic composition according to any one of the preceding claims, wherein the low-temperature catalyst is manganese monoxide, MnO.

6. The catalytic composition according to any one of the preceding claims, wherein in the catalytic mixture in a powdered form, comprising in weight-% with regard to the total mass of the catalytic mixture:
between 20 % to 65 % of the first catalyst having photocatalytic activity,
between 5 % to 55 % of the second catalyst having low-temperature activity,
being different to the first catalyst, and
between 30 % to 70 % of an adsorbent.

7. The catalytic composition according to any one of the preceding claims,
wherein the catalytic mixture in a powdered state comprises
between 27 % and 30 % of a first catalyst having photocatalytic activity,
between 11 % and 17 % of the second catalyst, being a low-temperature catalyst, and
between 55 % and 59 % of an adsorbent, based on the total mass of the catalytic mixture, in particular, being a powdered catalytic mixture comprising in weight-% in regard to its total mass
between 27 % and 30 % of photoactivated TiO₂,
between 11 % and 17 % of MnO,
between 55 % and 59 % of zeolite, in particular, hydrophilic zeolite of type A, and
between 0.1 % to 2 % based on the total amount of the catalytic mixture of carbon nanotubes.

8. A method for producing a catalytic composition containing a first catalyst having photocatalytic activity, a second catalyst different to the first catalyst being a low-temperature catalyst, an adsorbent present in form of a catalytic mixture, and carbon nanotubes comprising the steps of
a) Preparing a catalytic mixture composed of the first catalyst, the second catalyst and the adsorbent, whereby each of the components are provided in a powdered form, including mingling the same to obtain particles of the catalytic mixtures;
b) Admixing the particles of the catalytic mixture obtained in step a) with carbon nanotubes to obtain the catalytic composition.

9. The method according to claim 9, wherein the admixing of the carbon nanotubes with the particles of the catalytic mixture is mingling the components in powdered dry form.

10. The method according to claim 8, wherein a mixture of the carbon nanotubes with the catalytic mixture is a two-step mixing with a first mixing of the nanotubes with the catalytic mixture in dry form, and, subsequent solvent addition under agitation and heat, followed by evaporation of the solvent to obtain the catalytic composition.

11. The method according to any one of claims 8 to 10 for preparing a catalytic composition according to any one of claims 1 to 7.

12. A catalytic composition obtainable by a method according to any one of claims 8 to 11.

13. Catalytic device comprising the catalytic composition according to any one of claims 1 to 7 or 12 optionally coated on a carrier.

14. Gas depolluting apparatus comprising a catalytic device according to claim 13 and/or a catalytic composition according to any one of claims 1 to 7 or 12, wherein the catalytic device or the catalytic composition is at least partially provided with a designated flow path of the gas to be depolluted.

15. The use of a catalytic composition according to any one of claims 1 to 7 or 12 or obtainable by a method according to any one of claims 8 to 11, i) in gas depollution apparatus, in particular, for depollution of gaseous or volatile chemical contaminants and/or ii) in the treatment of gas containing biological contaminants.
